# EUROPEAN PATENT APPLICATION

(11) **EP 4 850 626 A1**
(43) Date of publication of application: **09.12.2026**
(21) Application number: 26181809.0
(22) Date of filing: 29.05.2026
(51) Int. Cl.: A61B 34/00, A61B 34/10, A61B 34/20, A61B 90/00

(54) **ADAPTIVE RENDERING FOR ENHANCED VISUALIZATION OF SURGICAL INSTRUMENTS AND IMPLANTS**

(30) Priority: 05.06.2025 US 202563818259 P
(71) Applicant: Stryker European Operations Limited, Carrigtwohill, Co. Cork T45 HX08 (IE)
(72) Inventor: Dube, Saurabh Matacharan, 400002 Mumbai (IN); Hertel, Julius Maximilian, 79114 Freiburg (DE); Martins, Maria Manuel Carvalho de Freitas, 68420 Eguisheim (FR)
(74) Representative: Schott, Jakob Valentin

(57) **Abstract**

Systems and methods for overlaying representations of a tracked object on a representation of a patient are provided. The tracked object may include at least one of a surgical instrument and an implant. The system includes a localizer, a display, and a controller in communication with the localizer and the display. The controller is configured to receive volumetric image data, determine a pose of the tracked object, and determine a pose of a view plane. The controller further causes the display to present a slice representation of the patient based on the pose of the view plane, determines a spatial relationship between the tracked object and the view plane, and identifies first and second portions of the tracked object that are on opposite sides of the view plane. Portions of the representation of the tracked object are overlaid on the slice representation of the patient based on the spatial relationship, with the portions displayed according to different visual characteristics.

## Description

### RELATED APPLICATIONS

The present application claims priority to and all the benefits of U.S. Provisional Patent Application No. 63/818259, filed on June 5, 2025, the entire contents of which are expressly incorporated herein by reference.

### BACKGROUND

Surgical navigation systems assist users in tracking/locating objects in an operating room. For instance, navigation systems assist surgeons in placing surgical instruments and/or implants relative to a patient's anatomy. Typically, the instrument and the anatomy are tracked together with their relative movement shown on a display. Often the navigation system includes tracking devices attached to the object being tracked, and a localizer determines positions of the tracking devices to determine a position and/or orientation of the object. The navigation system then monitors movement of the objects via the tracking devices, such as by determining the location of these tracking devices relative to a coordinate system associated with the localizer. During surgery, a surgeon often uses instruments that are inserted into the body of a patient. Once inside the patient's body, the surgeon loses vision of the tip of the instrument. In order to help the surgeon navigate the instrument in such a case, a surgical navigation system can be used to track the instrument and provide visual, tactile, and/or acoustic guidance to the surgeon. One way to track the instrument is to attach a tracker onto the instrument. A camera in the operating room detects the tracker and generates data that is used to calculate the position of the tracker and, therefore, the instrument. Commonly, the patient is also tracked, which enables calculating the pose of the instrument relative to the patient.

In many cases, representations of the instruments and/or implants are shown overlaid onto representations of the patient. For example, a slice image of the patient may be shown on a display, and a two/three-dimensional shape representative of the instrument and/or implant may be overlaid onto the slice image to illustrate a pose of the instrument and/or implant relative to the patient. Displaying these representations, however, may have certain limitations.

### SUMMARY

According to a first aspect, a surgical navigation system is provided. The surgical navigation system includes a localizer configured to track movement of a tracked object relative to a patient, a display configured to present a representation of the tracked object relative to a representation of the patient, and a controller in communication with the localizer and the display. The controller is configured to receive volumetric image data representative of the patient and defined relative to a first coordinate system, determine a pose of the tracked object relative to a second coordinate system based on signals received from the localizer, and register the first coordinate system to the second coordinate system. The controller is further configured to determine a pose of a view plane relative to the second coordinate system, cause the display to present a slice representation of the patient based on the pose of the view plane, determine a spatial relationship between the tracked object and the view plane, and identify a first portion of the tracked object that is on a first side of the view plane and a second portion of the tracked object that is on a second side of the view plane based on the spatial relationship. The controller is further configured to cause the display to present the representation of the tracked object on the slice representation of the patient such that the first portion and the second portion are visually distinguished from one another based on the spatial relationship relative to the view plane.

According to a second aspect, a surgical navigation system is provided. The surgical navigation system includes a localizer configured to track movement of a tracked object relative to a patient, a display configured to present a representation of the tracked object relative to a representation of the patient, and a controller in communication with the localizer and the display. The controller is configured to receive volumetric image data representative of the patient and defined relative to a first coordinate system, determine a pose of the tracked object relative to a second coordinate system based on signals received from the localizer, and register the first coordinate system to the second coordinate system. The controller is further configured to determine a pose of a view plane relative to the second coordinate system, cause the display to present a slice representation of the patient based on the pose of the view plane, determine a spatial relationship between the tracked object and the view plane, and identify a first portion of the tracked object that is on a first side of the view plane and a second portion of the tracked object that is on a second side of the view plane based on the spatial relationship. The controller is configured to cause the display to overlay a first portion of the representation of the tracked object on the slice representation of the patient based on the spatial relationship and according to a first visual characteristic corresponding to the first portion, and cause the display to overlay a second portion of the representation of the tracked object on the slice representation of the patient based on the spatial relationship and according to a second visual characteristic that is different from the first visual characteristic, the second portion corresponding to the second portion of the tracked object.

According to a third aspect, a surgical navigation system is provided. The surgical navigation system includes a localizer configured to track movement of a tracked object relative to a patient, the tracked object including at least one of a surgical instrument and an implant, a display configured to present a representation of the tracked object relative to a representation of the patient, and a controller in communication with the localizer and the display. The controller is configured to receive volumetric image data representative of the patient and defined relative to a first coordinate system, determine a pose of the tracked object relative to a second coordinate system based on signals received from the localizer, and register the first coordinate system to the second coordinate system. The controller is further configured to determine a pose of a view plane relative to the second coordinate system, cause the display to present a slice representation of the patient based on the pose of the view plane, determine a spatial relationship between the tracked object and the view plane, and identify a first portion of the tracked object that is on a first side of the view plane and a second portion of the tracked object that is on a second side of the view plane based on the spatial relationship. The controller is configured to cause the display to overlay a first portion of the representation of the tracked object on the slice representation of the patient based on the spatial relationship, and cause the display to hide a second portion of the representation of the tracked object such that the second portion is not overlaid on the slice representation.

According to a fourth aspect, a surgical navigation system is provided. The surgical navigation system includes a localizer configured to track movement of a surgical instrument relative to a patient, a display configured to present a representation of the surgical instrument relative to a representation of the patient, and a controller in communication with the localizer and the display. The controller is configured to receive volumetric image data representative of the patient and defined relative to a first coordinate system, determine a pose of the surgical instrument relative to a second coordinate system based on signals received from the localizer, and register the first coordinate system to the second coordinate system. The controller is configured to determine a pose of a view plane relative to the second coordinate system, cause the display to present a slice representation of the patient based on the pose of the view plane, determine a spatial relationship between the surgical instrument and the view plane, and identify a first portion of the surgical instrument that is on a first side of the view plane and a second portion of the surgical instrument that is on a second side of the view plane based on the spatial relationship. The controller is configured to cause the display to overlay a first portion of the representation of the surgical instrument on the slice representation of the patient based on the spatial relationship and according to a first visual characteristic, and to cause the display to overlay a second portion of the representation of the surgical instrument on the slice representation of the patient based on the spatial relationship and according to a second visual characteristic that is different from the first visual characteristic. The first portion of the representation corresponds to the first portion of the surgical instrument, and the second portion of the representation corresponds to the second portion of the surgical instrument.

According to a fifth aspect, a surgical navigation system is provided. The surgical navigation system includes a localizer configured to track movement of a surgical instrument relative to a patient, a display configured to present a representation of the surgical instrument relative to a representation of the patient, and a controller in communication with the localizer and the display. The controller is configured to receive volumetric image data representative of the patient and defined relative to a first coordinate system, determine a pose of the surgical instrument relative to a second coordinate system based on signals received from the localizer, and register the first coordinate system to the second coordinate system. The controller is further configured to determine a pose of a view plane relative to the second coordinate system, cause the display to present a slice representation of the patient based on the pose of the view plane, determine a spatial relationship between the surgical instrument and the view plane, and identify a first portion of the surgical instrument that is on a first side of the view plane and a second portion of the surgical instrument that is on a second side of the view plane based on the spatial relationship. The controller is configured to cause the display to overlay a first portion of the representation of the surgical instrument on the slice representation of the patient based on the spatial relationship, and cause the display to hide a second portion of the representation of the surgical instrument such that the second portion is not overlaid on the slice representation.

According to a sixth aspect, a surgical navigation system is provided. The surgical navigation system includes a localizer configured to track movement of a surgical instrument relative to a patient, a display configured to present a representation of an implant coupled to the surgical instrument relative to a representation of the patient, and a controller in communication with the localizer and the display. The controller is configured to receive volumetric image data representative of the patient and defined relative to a first coordinate system, determine a pose of the surgical instrument relative to a second coordinate system based on signals received from the localizer, register the first coordinate system to the second coordinate system, determine a pose of a view plane relative to the second coordinate system, and cause the display to present a slice representation of the patient based on the pose of the view plane. The controller is further configured to determine a spatial relationship between the implant and the view plane, and identify a first portion of the implant that is on a first side of the view plane and a second portion of the implant that is on a second side of the view plane based on the spatial relationship. The controller is configured to cause the display to overlay a first portion of the representation of the implant on the slice representation of the patient based on the spatial relationship and according to a first visual characteristic, and cause the display to overlay a second portion of the representation of the implant on the slice representation of the patient based on the spatial relationship and according to a second visual characteristic that is different from the first visual characteristic. The first portion of the representation corresponds to the first portion of the implant, and the second portion of the representation corresponds to the second portion of the implant.

According to a seventh aspect, a surgical navigation system is provided. The surgical navigation system includes a localizer configured to track movement of a surgical instrument relative to a patient, a display configured to present a representation of an implant coupled to the surgical instrument relative to a representation of the patient, and a controller in communication with the localizer and the display. The controller is configured to receive volumetric image data representative of the patient and defined relative to a first coordinate system, determine a pose of the surgical instrument relative to a second coordinate system based on signals received from the localizer, and register the first coordinate system to the second coordinate system. Further, the controller is configured to determine a pose of a view plane relative to the second coordinate system, cause the display to present a slice representation of the patient based on the pose of the view plane, determine a spatial relationship between the implant and the view plane, and identify a first portion of the implant that is on a first side of the view plane and a second portion of the implant that is on a second side of the view plane based on the spatial relationship. The controller is configured to cause the display to overlay a first portion of the representation of the implant on the slice representation of the patient based on the spatial relationship, and cause the display to hide a second portion of the representation of the implant such that the second portion is not overlaid on the slice representation.

Also provided are: computer-implemented methods of operating the surgical navigation system of any preceding aspect; and non-transitory computer readable media (or computer program products) comprising instructions, which when executed by one or more processors, are configured to execute the capabilities of the surgical navigation system or controller of any preceding aspect. Any of the above aspects can be combined in part or in whole with any other aspect. Any of the above aspects, whether combined in part or in whole, can be further combined with any of the following implementations in full or in part.

Depending on the implementation, portions of a representation of a tracked object may be visually distinguished from one another based on a spatial relationship relative to a view plane. The visual distinction may be achieved by applying different visual characteristics and/or by controlling visibility of the portions. The visual characteristics may include, for example, at least one of: an opacity of the corresponding portion of the representation, a transparency of the corresponding portion of the representation, a color of the corresponding portion of the representation, an outline of the corresponding portion of the representation, and a shading of the corresponding portion of the representation. In some implementations, the first visual characteristic may be a first color and the second visual characteristic may be a second color different from the first color. In another example, the first visual characteristic may be a first opacity and the second visual characteristic may be a second opacity different from the first opacity. In yet another example, the first visual characteristic may correspond to a filled representation and the second visual characteristic may correspond to an outline representation. In some implementations, one portion of the representation may be displayed while another portion is hidden such that it is not overlaid on the slice representation.

The tracked object may include a surgical instrument, an implant, or a combination thereof, such as an implant supported by or coupled to the surgical instrument. The surgical navigation system may be used with various types of tracked objects, some of which may include a critical portion, such as a cutting edge or functional feature. In some implementations, the controller may be configured to select a visual characteristic or visibility state of a portion of the representation based on whether the portion includes a critical portion of the tracked object. Additionally or alternatively, the controller may be configured to determine whether a portion of the tracked object includes a critical portion and cause the display to present a representation of the critical portion on the slice representation of the patient according to a third visual characteristic that is different from other visual characteristics. In some implementations, the representation of the critical portion may be overlaid even when another portion of the representation is hidden.

In some implementations, the view plane may be realized as a first view plane and the slice representation may be realized as a first slice representation of the patient. The controller may be configured to determine a pose of a second view plane relative to the coordinate system, the second view plane being orthogonal to the first view plane, and cause the display to present a second slice representation of the patient based on the pose of the second view plane, the first and second slice representations being orthogonal representations of the patient. The controller may be further configured to determine a spatial relationship between the tracked object and the second view plane and identify portions of the tracked object on opposite sides of the second view plane. Based on the spatial relationship, the controller may cause the display to present corresponding portions of the representation of the tracked object on the second slice representation according to different visual characteristics and/or visibility states. In some implementations, a portion of the representation corresponding to one side of the second view plane may be overlaid while a portion corresponding to another side is hidden. In some implementations, the first slice representation may be an axial slice representation of the patient and the second slice representation may be a sagittal slice representation of the patient.

In some implementations, the tracked object may include both a surgical instrument and an implant. The controller may be configured to determine a spatial relationship between the implant and the view plane and cause the display to present a representation of the implant on the slice representation of the patient based on the spatial relationship. The controller may be configured to identify portions of the implant on opposite sides of the view plane and cause the display to present corresponding portions of the representation of the implant according to different visual characteristics and/or visibility states. In some implementations, a first portion of the representation of the implant may be overlaid on the slice representation and a second portion may be displayed with a different visual characteristic or may be hidden. The portions of the representation may correspond to respective portions of the implant.

In some implementations, the representation of the tracked object may be based on a three-dimensional model that is representative of the tracked object, such as a model of a surgical instrument, an implant, or both. In some implementations, the controller may be configured to determine the pose of the view plane based on a position of the tracked object, including at least one of a position of the surgical instrument and a position of the implant.

The operating characteristics of the surgical navigation system may be embodied in various forms. In some implementations, a method of overlaying representations of a tracked object on a representation of a patient is provided, the method including operations performed by the controller as described above. In some implementations, a non-transitory computer-readable medium having instructions stored thereon is provided, the instructions being executable by a controller to cause the controller to perform the operations described above.

### BRIEF DESCRIPTION OF THE DRAWINGS

Advantages of the present disclosure will be readily appreciated as the same becomes better understood by reference to the following detailed description when considered in connection with the accompanying drawings.
Figure 1 shows an exemplary configuration of a surgical suite for performing a medical procedure on a patient according to one implementation.
Figure 2 shows a graphical user interface of a surgical navigation system according to one implementation.
Figure 3 shows a flowchart describing a method of overlaying an instrument representation on a slice representation of a patient according to one implementation.
Figure 4 shows an instrument positioned relative to a patient and view planes representation on a slice representation of a patient according to the method of Figure 3.
Figures 5A and 5B show a spatial relationship between the instrument and the first and second view planes of Figure 4.
Figures 6A and 6B show the GUI of Figure 2 along with an instrument representation overlaid on slice representations of a patient according to the spatial relationship shown in Figures 5A and 5B and the method of Figure 3.
Figures 7A and 7B show a spatial relationship between an implant and first and second view planes.
Figures 8A and 8B show the GUI of Figure 2 along with an implant representation overlaid on slice representations of a patient according to one implementation.

### DETAILED DESCRIPTION

In the following description, exemplary embodiments will be explained with reference to the drawings. The same reference numerals will be used to denote the same or similar structural features.

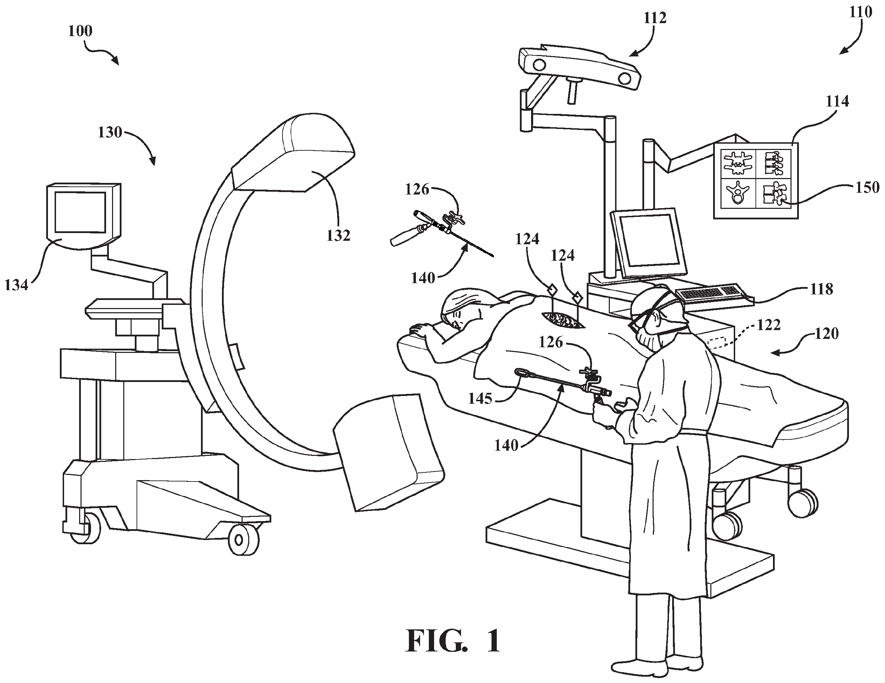

Referring to FIG. 1, an exemplary configuration of a surgical suite for performing a medical procedure on a patient using a surgical system 100 is shown. The surgical system 100 may include a surgical navigation system 110, a surgical imaging system 130, and at least one surgical instrument 140. The surgical navigation system 110 may include a localizer 112, a display 114 configured to display a graphical user interface (GUI) 150, a user input device 118, a navigation computer 120, and a controller 122 to facilitate communication between the navigation computer 120, the remainder of the navigation system 110, and optionally other elements of the system 100. Although not shown, the navigation computer 120 may include a separate processor, a memory device, and/or a storage device. The navigation computer 120 may be a personal computer, laptop computer, tablet computer or any other suitable computing device. The navigation computer 120 may include surgical navigation software including one or more modules and/or operating instructions related to the operation of the surgical navigation system 110 and to implement the various routines, functions, or methods disclosed herein. Further, the controller 122 may be part of the navigation computer 120, or the controller 122 may be implemented by multiple computing devices, such as the navigation computer 120 and a cloud computing device. In some implementations, the controller 122 includes a cloud computing device which communicates with the navigation computer 120 (e.g. the processor of the navigation computer 120).

The display 114 may be configured to display the GUI 150 as well as patient images (e.g., pre-operative patient images or intraoperative patient images). The pre-operative images may be uploaded to the surgical navigation system 110 prior to the surgical procedure. A user such as a medical professional may interact with the GUI 150 via user input devices 118, touch input, or other suitable means. The display 114 of the surgical navigation system 110 may be configured to display various prompts or data entry boxes. For example, the display 114 may be configured to display a text box or prompt that allows the user to manually enter or select the type of surgical procedure to be performed.

The display 114 may be further configured to display a surgical plan for a medical procedure overlaid on the patient images. The surgical plan may include the surgical pathway for executing the medical procedure, planned trajectory, orientation, and/or position for the medical instrument and/or implant during the medical procedure. The surgical plan may also include a pose of an implant or medical device to be inserted during the medical procedure overlaid onto the patient data or image. It is contemplated that the surgical navigation system 110 may be configured to display and/or project a holographic image of surgical pathway for executing the medical procedure or planned trajectory or orientation for the medical instrument during the medical procedure. This may include projecting the surgical pathway onto the patient or other surface in the operating room. It may also include a projection of the surgical pathway onto the head unit worn by the user, such as a lens, shield, or glasses of the head unit. An exemplary configuration of the surgical navigation system 110 including a display worn by the user to display the target trajectory and/or target location is disclosed in International Publication No. WO/2018/203304 A1, the entirety of which is hereby incorporated by reference.

In some implementations, the display 114 may be configured as an extended reality device configured to execute any of the graphical functions described herein. The extended reality device can be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the user. The real-world views may be views may be those acquired directly by the eyes of the user or may be a real-world video stream captured by one or more cameras of the extended reality device. When a head-mounted device is utilized, the head-mounted device may comprise a transparent lens or one or more display screens positioned directly in front of the eyes of the user to display the computer-generated graphics relative to the real-world views.

The GUI 150 may be configured to allow the user to input or enter patient data or modify the surgical plan. The patient data, in addition to the patient images, may include additional information related to the type of medical procedure being performed, the patient's anatomical features, the patient's specific medical condition, and/or operating settings for the surgical navigation settings. For example, in performing a spinal fusion procedure, the user may enter information via the user input device 118 and/or the GUI 150 related to the specific vertebra or vertebra on which the medical procedure is being performed. The user may also input various anatomical dimensions related to the vertebrae and/or the size and shape of a medical device or implant to be inserted during the medical procedure. The user input device 118 and/or the GUI 150 may also be configured to allow the user to select, edit or manipulate the patient data. For example, the user may identify and/or select anatomical features from the patient data. This may include selecting the surgical site, such as selecting the vertebra and/or specific area/level on the vertebra where the medical procedure is to be performed.

The surgical navigation system 110 may be configured to utilize segmentation to facilitate various features of surgical navigation, such as tool guidance and the generation of alert zones of interests around critical anatomical features. These critical anatomical features may include, cortical walls, nerves, blood vessels or similar critical anatomical structures. The alert zones may be defined by one or more virtual boundaries. The user may also provide input to the user input device 118 or to the GUI 150 to identify additional critical anatomical features and/or alert zones in addition to what was suggested by the controller 122 or wish to edit alert zones and/or virtual boundaries generated by the controller 122. The user may also provide input to the user input device 118 or to the GUI 150 to select and/or input a target location, target trajectory, target depth or similar feature of the surgical pathway to help guide the user in performing the medical procedure. The input to the user input device 118 or to the GUI 150 may be provided to select the surgical instrument to be used, to select the device and/or implant to be inserted, to select a planned pose where the device or implant is to be placed within the patient, and to allow the user to select the parameters of the implant to be inserted, such as a length and/or diameter of a screw to be inserted.

The surgical system 100 may also include an imaging system 130 in communication with the surgical navigation system 110. The imaging system 130, such as CT or MRI imaging device, may perform preoperative imaging and/or intraoperative imaging. If the imaging system 130 is a CT imaging device, the imaging system 130 may generate CT image data. The imaging system 130 may include a scanner 132 and an imager display 134. The scanner 132 may be utilized to take an image of the patient and display it on the imager display 134. For example, the scanner 132 may include a C-arm configured to be rotated about the patient to produce a plurality of images of the patient. The imaging system 130 may also include a processor (not shown) including software, as is known by those skilled in the art, which is capable of taking the plurality of images captured by the scanner 132 and producing a two-dimensional image and/or a three-dimensional model of at least a portion of the patient. The imager display 134 may be configured to display the resulting two-dimensional image and/or three-dimensional model.

The imaging system 130 may also be in communication with the controller 122 of the surgical navigation system 110. The imaging system 130 may be configured to communicate via a wired and/or a wireless connection with the controller 122. For example, the imaging system 130 may be configured to provide pre-operative and/or intra-operative image data, such as the resulting 2D image and/or 3D model of the patient, to the controller 122 to provide the resulting 2D image and/or 3D model to the display 114. If the imaging system 130 is a CT imaging device, the imaging system 130 may provide the controller 122 with CT image data.

The surgical navigation system 110 may track the pose of the patient based on a patient tracker 124 coupled to or near the patient. The patient may also be tracked according to machine vision techniques such that the pose of the patient may be determined by the localizer 112 but without the patient tracker 124. Further, the surgical system 100 may include a surgical instrument 140. Similar to patient tracking, the surgical navigation system 110 may include an instrument tracker 126 configured to be coupled to the instrument 140 to allow the pose of the instrument 140 to be tracked by the surgical navigation system 110. Similar to patient tracking, the instrument 140 may also be tracked according to machine vision techniques such that the pose of the instrument 140 may be determined by the localizer 112 without the instrument tracker 126. In implementations that utilize the tracking devices 124, 126, the tracking devices 124, 126 may include a plurality of markers that are capable of being identified and/or tracked by the surgical navigation system 110. Reliable tracking of surgical instrument pose relative to patient pose during the execution of surgical procedures (e.g., for helping the user/surgeon follow the planned surgical pathway and/or to avoid critical anatomical structures) is of the utmost importance. Furthermore, providing feedback and/or notifying the user executing the procedure when the surgical instrument becomes misaligned with the surgical pathway and/or is at risk of impinging on a critical anatomical structure is of similar importance.

The navigation system 110 may utilize the localizer 112 to track the instrument 140, an implant 145, the implant 145 coupled to the surgical instrument 140, the surgical robot, and/or other elements of the surgical system 100. As used herein, the term "tracked object" refers to any object whose pose is determined by the surgical navigation system, including but not limited to the surgical instrument 140, the implant 145, or the implant 145 coupled to the surgical instrument 140. The localizer 112 may also be used to track the patient. The localizer 112 may track the poses of the instrument 140, the surgical robot, other elements of the surgical system 100, and/or the patient relative to a localizer coordinate system. For example, the localizer 112 may include one or more sensors for tracking the tracking device 126 coupled to the surgical instrument 140 and the patient tracker 124 coupled to the patient. The sensors may include cameras, such as CCD cameras, CMOS cameras, and/or optical image cameras, magnetic sensors, radio frequency sensors, or any other sensor adapted to detect and/or sense the position of the trackers 124, 126. Description of a suitable localizer, and the various localizers that it can utilize may be found in U.S. Patent Publication No. 2017/0333137, which is hereby incorporated by reference in its entirety.

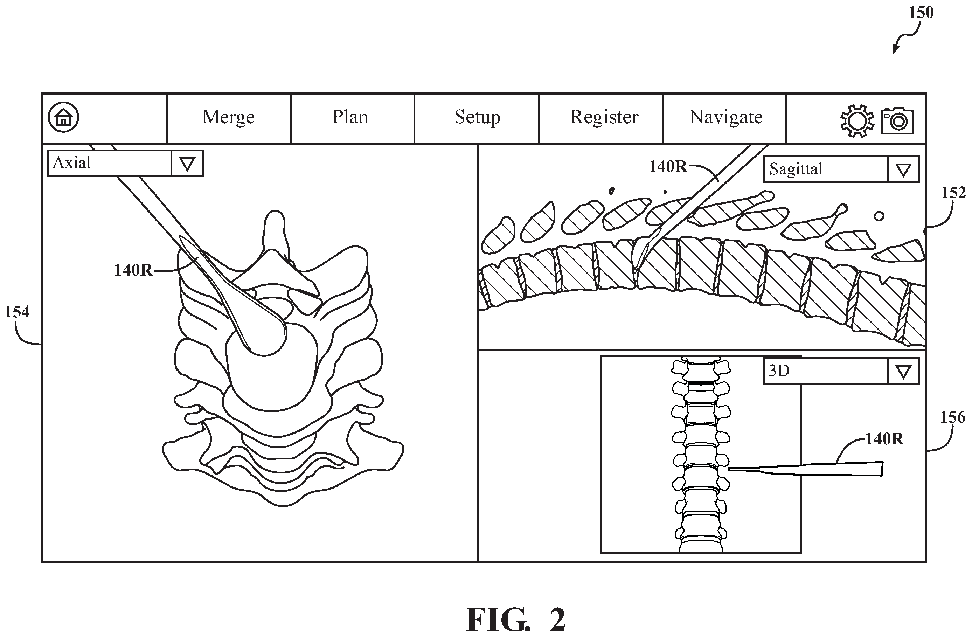

Referring to FIG. 2, an exemplary configuration of the graphic user interface (GUI) 150 of the surgical navigation system 110 is illustrated. The GUI 150 may be controlled by the controller 122 and/or receive inputs via a touch screen of the display 114 of the surgical navigation system 110, an interactable object in a virtual/augmented reality space, or any suitable alternative. In some implementations, the GUI 150 may be implemented as an interactable object in a virtual/augmented reality space.

In the illustrated implementation, the GUI 150 includes a first window 152, a second window 154, and a third window 156, each of which contain a view of a patient image. In this implementation, the first window 152 contains a sagittal view of the patient image, the second window 154 contains an axial view of the patient image, and the third window 156 contains a perspective view of a 3D model of the patient. The GUI 150 is shown operating in a navigation mode, but the GUI may also operate in other modes, such as a planning mode and a registration mode. The methods and functions of the system 100 are described with reference to the illustrated implementation in which the GUI 150 is operating in the navigation mode, but may also be carried out while the GUI 150 is in the planning mode, the registration mode, or any other mode.

As described above, the display 114 is configured to display patient images (e.g., preoperative patient images or intraoperative patient images) via the GUI 150. An entirety of the patient image, or a portion thereof, may be shown in the windows 152, 154, 156. The patient image may be a volumetric image, such as a CT image, and the windows 152, 154, 156 may each contain a "view" corresponding to the volumetric image. For example, each view may include at least a portion of a two-dimensional slice image derived from the volumetric image. Alternatively, the slice image may be a raw CT slice image upon which the volumetric image was generated. The views may alternatively include other two-dimensional images as long as a relationship between the two-dimensional images and the volumetric images is known. In Figure 2, the views shown in the first and second windows 152, 154 each include a portion of a slice image which corresponds to slices of the volumetric image, and the view shown in the third window 156 includes a portion of a 3D model created based on the volumetric image. As such, the memory device may contain at least one slice image. The pose of the slice image(s) relative to the image coordinate system (and thus the volumetric image) may be known. The slice image(s) may be CT slice images, digitally reconstructed radiographs (DRRs), or other suitable alternatives.

A virtual representation 140R, also called an instrument representation 140R, of at least part of the surgical instrument 140 may be overlaid onto the patient image in at least one of the windows 152, 154, 156 based on the tracked pose of the instrument 140 relative to the patient. The instrument representation 140R may be based on a three-dimensional instrument model that is representative of the surgical instrument 140. As described below, the instrument representation 140R may be overlaid on the slice representation of the patient in manners which give the user a more accurate view of a spatial relationship between the instrument 140 and the slice representation being shown via the GUI 150. More specifically, the instrument representation 140R may be overlaid on the slice representation such that the user can appreciate which portions of the instrument 140 are "in front" of the anatomy featured in the slice representation and which portions of the instrument 140 are "behind" of the anatomy featured in the slice representation. This advantageously provides the user with a more accurate view of the pose of the instrument relative to the patient.

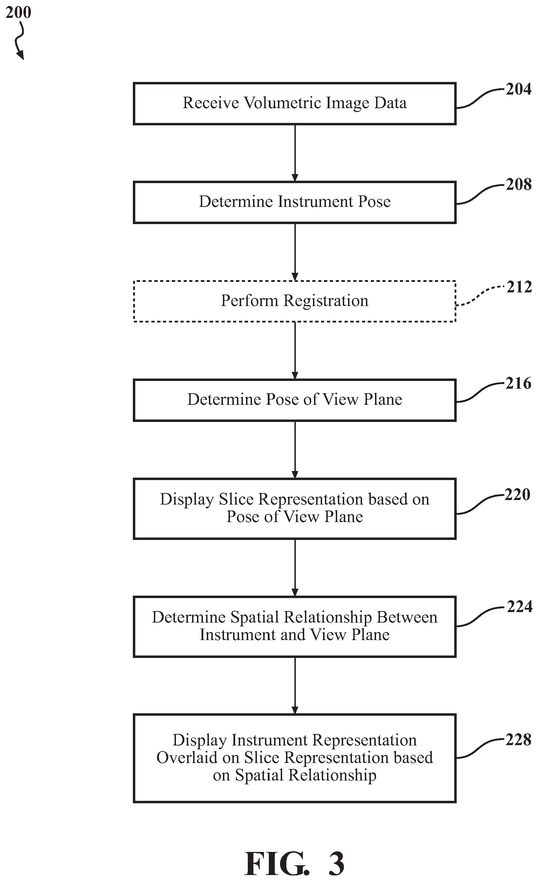

Referring to FIGS. 3, a flowchart illustrative of a method 200 for displaying representations of the instrument 140 relative to image data of the patient is shown. The method 200 is described as being carried out by the controller 122, but it is further contemplated to execute the method via at least one alternative computing device, such as a cloud computing device/environment. The controller 122 may also be used for part of the method 200, while the cloud computing device/environment may be used for the remainder of the method 200.

Starting with FIG. 3, a flowchart representative of the method 200 is shown. Starting at 204, the controller 122 receives volumetric image data. The volumetric image data is representative of at least a portion of the patient and may include CT image data, MRI image data, or other suitable forms of volumetric image data. The volumetric image data may be defined relative to a first coordinate system, such as an image coordinate system associated with the imaging system 130. Subsequently, at 208, the controller 122 determines a pose of the instrument 140. The pose of the instrument 140 may be determined relative to a second coordinate system, such as a localizer coordinate system associated with the localizer 112. This may include receiving signals from the localizer 112 that correspond to a pose of the instrument tracker 126. In implementations where the volumetric image data and instrument pose are defined/determined relative to different coordinate systems, the method 200 may include registering the coordinate systems to one another at 212. For example, the first coordinate system may be registered to the second coordinate system or vice versa. In a more specific example, the image coordinate system may be registered to the localizer coordinate system or vice versa. Any suitable method of registration may be used at 212. The method 200 then moves to 216.

After the volumetric image data and instrument pose are received and determined, and the coordinate systems are optionally registered to one another, the method 200 carries out steps 216 through 228 to cause the at least one of the displays 114, 134 to show the pose of the instrument 140 relative to the pose of the patient. The GUI 150 may be configured to show two-dimensional (i.e., slice) representations of the patient with the instrument representation 140R overlaid onto the slice representation of the patient. Further, the instrument representation 140R may be overlaid on the slice representation with an indication of which portions of the instrument 140 are in front of, rather than behind, the anatomy featured in the slice representation.

In order to accurately show which portions of the instrument 140 are on a first side (e.g., in front) of the slice representation and which portions are on a second side (e.g., behind) of the slice representation, the system 100 may start by determining a pose of a view plane that will define the pose of the slice representation at 216. For example, if the slice representation to be shown was an axial slice of a three-dimensional model of the patient, the view plane may be disposed axially relative to the patient and/or the model of the patient. In implementations where multiple slice representations of the patient are shown via the GUI 150, the controller 122 may determine poses of multiple view planes, such as two orthogonal view planes, at step 216. The view plane(s) may be defined such that axial and/or sagittal slice representations of the patient are shown via the GUI 150. The view plane(s) may be defined relative to the first or second coordinate system, or any other known coordinate system.

In some implementations, the view plane(s) may be defined based on a position or pose of the instrument 140 to ensure that the instrument representation 140R is overlaid on a slice representation that represents patient anatomy near the instrument 140. Alternatively or additionally, the view plane(s) may be defined based on a selected anatomical object of interest, such as a specific vertebra/vertebral level. The selection may be based on a user input to the GUI 150 and/or a surgical plan being carried out.

Once the controller 122 has determined the pose of the view plane (or view planes), the slice representation is shown on at least one of the displays 114, 134 based on the pose of the view plane at 220. Depending on the implementation, the slice representation may be a slice image included in the image data, a digitally reconstructed radiograph (DRR), or a slice of the volumetric model itself.

While the slice representation(s) of the patient are shown via the GUI 150, the system 100 may continue to track the pose of the instrument 140 to determine if any portion of the instrument 140 intersects the view plane (and the slice representation by extension). To this end, the controller 122 may determine a spatial relationship between the instrument 140 and the view plane at 224. This may include defining a first side of the view plane and a second side of the view plane, and identifying which portions of the instrument 140 are on the first side of the view plane versus the second side of the view plane. The sides of the view plane may be defined based on user input, surgical plan, or other suitable methods. Once the spatial relationship between the instrument 140 and the view plane has been determined, the controller 122 may cause at least one of the displays 114, 134 to overlay the instrument representation 140R on the slice representations based on the spatial relationship at 228. In other words, the controller 122 may cause the instrument representation 140R to be overlaid on the slice representation so as to give the user an idea of which portions of the instrument 140 are behind/in front of the anatomy included in the slice representation. This may include showing portions of the instrument representation 140R according to different visual characteristics, such as opacity, transparency, color, shade, fill, or other visual characteristics suitable for differentiating different portions of the instrument representation 140R. Additionally or alternatively, this may include showing the portion of the instrument representation 140R that corresponds to the portion of the instrument 140 which is on the first side ("in front of") of the view plane and slice representation, and hiding the portion of the instrument representation 140R that corresponds to the portion of the instrument 140 which is on the second side of ("behind") the view plane and slice representation.

During step 228 of the method 200, the controller 122 may cause the GUI 150 to overlay a first portion of the instrument representation 140R according to a first visual characteristic and a second portion of the instrument representation 140R according to a second visual characteristic. In a first example, the first visual characteristic is a first color and the second visual characteristic is a second color that is different from the first color. In a second example, the first visual characteristic is a first opacity and the second visual characteristic includes a second opacity different than the first opacity. In a third example, the first visual characteristic is a filled shape and the second visual characteristic is a shape outline. Other combinations are contemplated.

The instrument 140 may include a critical portion that is necessary or helpful to show at all times, such as a cutting edge of the instrument 140. As such, in some implementations, the controller 122 may be configured to determine whether the second portion of the instrument representation 140R includes any of the critical portion of the instrument 140, such as at 224 during the method 200. Then, as a result of this determination, the controller 122 may cause the GUI 150 to overlay a portion of the instrument representations 140R that corresponds to the critical portion of the instrument 140 on the slice representation of the patient according to a third visual characteristic that is different from the first visual characteristic and the second visual characteristic and based on the spatial relationship between the instrument 140 and the view plane, such as at 228 during the method 200. In an example, the controller 122 may first determine that half of the distal portion of the instrument 140 is on the second side of the view plane, subsequently determine that a portion of this half of the distal portion of the instrument 140 includes some of the cutting edge of the instrument 140. In response, the controller 122 may display the instrument representation 140R in three ways. First, the portion of the instrument 140 that is on the first side of the view plane may be overlaid on the slice representation according to a first visual characteristic, such as near-opaque. Second, the portion of the instrument 140 that is on the second side of the view plane but does not include the critical portion may be overlaid according to a second visual characteristic, such as translucent. Alternatively, the second portion may be hidden from view and not overlaid on the slice representation. Third, the critical portion of the instrument 140 that is on the second side of the view plane may be overlaid on the slice representation according to a third visual characteristic, such as a dotted line. In implementations where the second portion of the instrument representation 140R is hidden and a part of the second portion includes some/all of the critical portion, this part of the second portion may be unhidden.

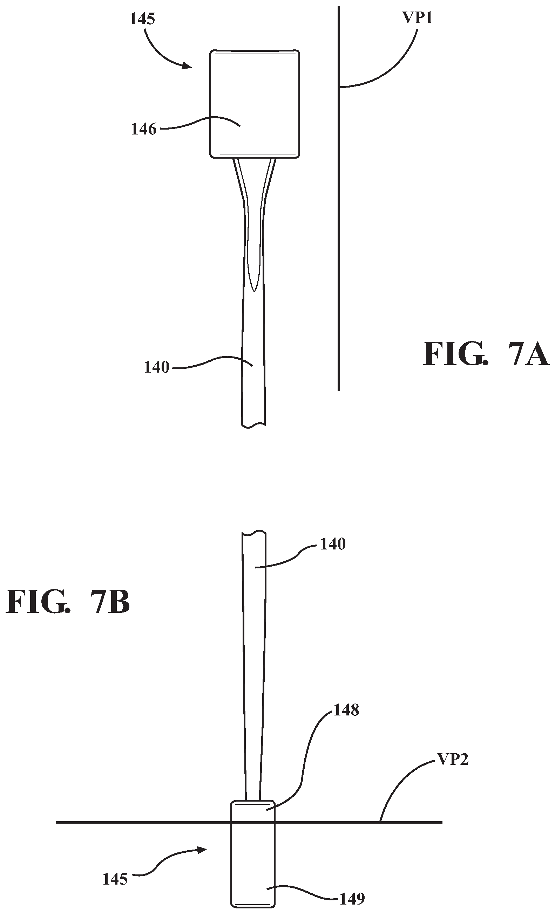

Additionally or alternatively, the instrument 140 may include an implant 145, and the controller 122 may be configured to cause the display to overlay a representation of the implant 145R, also called an implant representation 145R, on the slice representation of the patient based on the spatial relationship between the instrument 140 (and/or the implant 145) and the view plane. Similar to the instrument representation 140R, the implant representation 145R may be based on a three-dimensional implant model that is representative of the implant 145. In one example, the instrument 140 may be a trial inserter having a trial implant coupled to the remainder of the instrument 140 or an implant inserter (e.g., a cage inserter) having an implant coupled thereto. Regardless of the form or type of instrument which includes the implant 145, the pose of the implant 145 may be tracked based on the pose of the instrument 140 (and/or the pose of the implant 145 itself) and the controller 122 may be configured to identify a first portion of the implant 145 that is on the first side of the view plane and a second portion of the implant 145 that is on the second side of the view plane based on the spatial relationship. For example, at 224 during the method 200. Afterwards, the controller 122 may cause the GUI 150 to overlay a first portion 146R of an implant representation 145R on the slice representation of the patient according to the first visual characteristic and overlay a second portion 147R of the implant representation 145R on the slice representation of the patient according to the second visual characteristic, both based on the spatial relationship (see FIGS. 7A and 7B). For example, at 228 during the method 200.

Like the instrument 140, the implant 145 may include a critical portion that is necessary or helpful to show at all times. As such, it is also contemplated to display critical portion(s) of the implant representation 145R in ways similar to those described in reference to critical portions of the instrument representation 140R. The critical portions of the implant 145 may include an outer profile of the implant 145, a portion of the implant 145 that is coupled to the instrument 140, or any other portion of the implant 145 that is determined to be critical to the user.

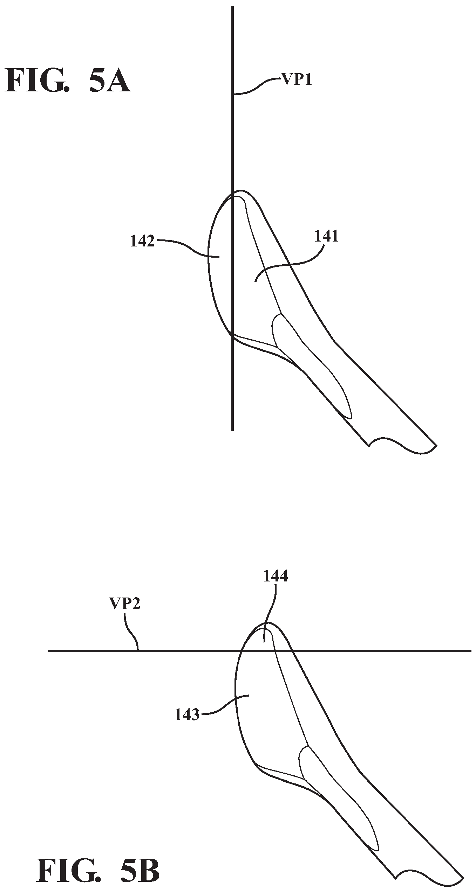

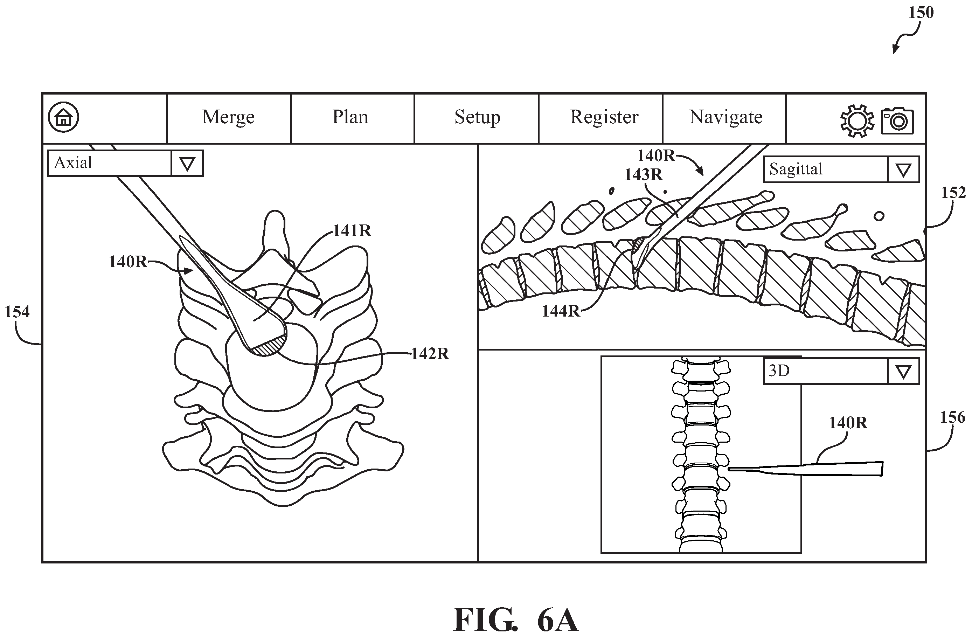

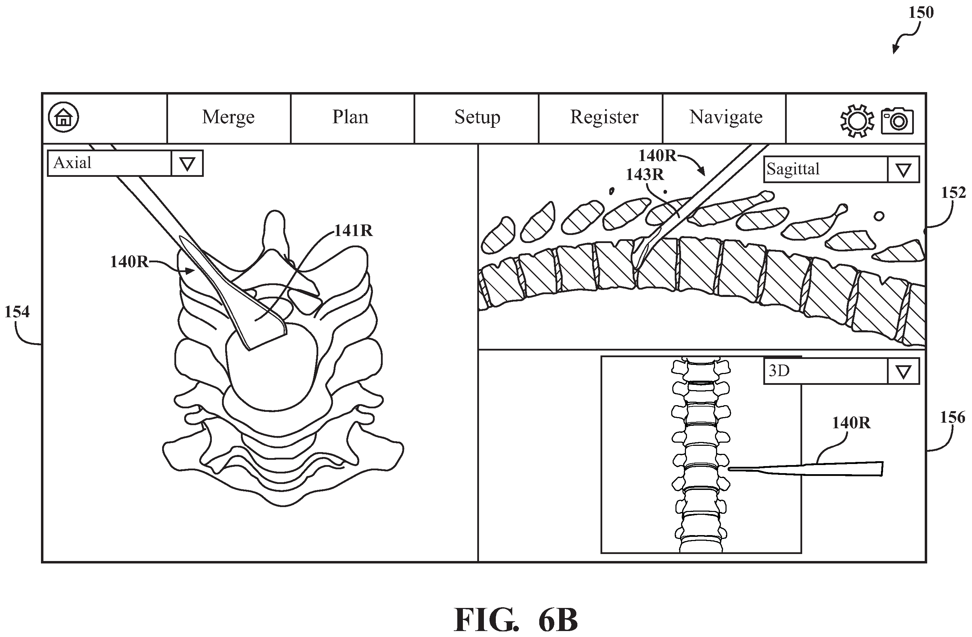

As described above, the controller 122 may determine poses of multiple view planes, such as two orthogonal view planes, at step 216 such that multiple slice representations of the patient may be shown via the GUI 150. To this end, the controller 122 may determine a pose of a second view plane relative to the second coordinate system, such as at 216 during the method. Based on the pose of the first and second view planes, the controller 122 may cause the GUI 150 to show first and second slice representations of the patient based on the poses of the first and second view planes, such as at 220 during the method 200. The controller 122 may then determine the spatial relationship between the surgical instrument 140 and both of the first and second view planes, such as at 224 during the method. In addition to identifying the first and second portions of the instrument 140 as described above in reference to step 224, this may further include identifying a third portion of the surgical instrument 140 that is on a first side of the second view plane and a fourth portion of the surgical instrument 140 that is on a second side of the second view plane, both based on the spatial relationship between the surgical instrument 140 and the second view plane (see FIG. 5B). The controller 122 may cause the GUI 150 to overlay a third portion of the instrument representation 140R on the second slice representation of the patient based on the spatial relationship and according to the first visual characteristic, and cause the GUI 150 to overlay a fourth portion of the instrument representation 140R on the second slice representation of the patient based on the spatial relationship and according to the second visual characteristic that is different from the first visual characteristic (see FIGS. 6A and 6B). The third portion of the representation 140R may correspond to the third portion of the surgical instrument 140 and the fourth portion of the representation 140R may correspond to the fourth portion of the surgical instrument 140.

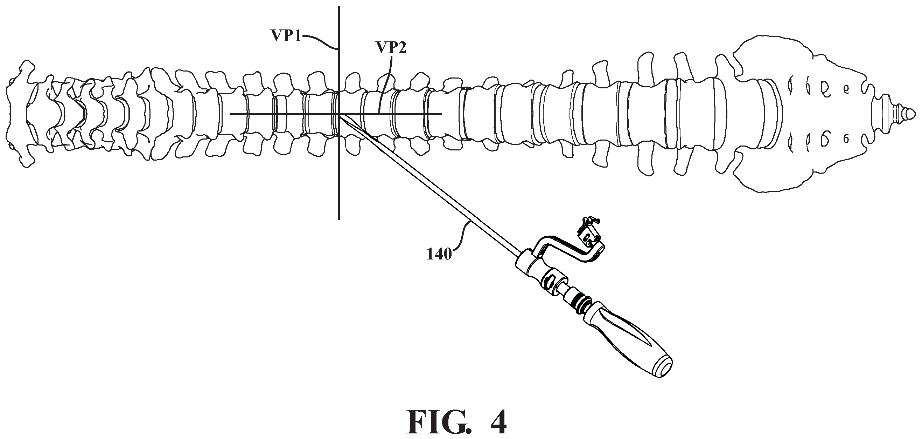

Referring to FIGS. 4-6B, an example overlaying the instrument 140 on the slice representation of the patient according to the method 200 is illustrated. Starting with FIG. 4, the instrument 140 is shown relative to the spine of the patient. In the illustrated example, the T6 vertebra has been selected (e.g., based on a surgical plan or user input), and a first view plane VP1 and a second view plane VP2 are shown, each of which correspond to the T6 vertebra. More specifically, the first view plane VP1 corresponds to an axial view of the T6 vertebra and the second view plane VP2 corresponds to a sagittal view of the T6 vertebra. Moving to FIGS. 5A and 5B, the spatial relationship between the instrument 140 and the first and second view planes VP1, VP2 from FIG. 4 is illustrated in more detail. In FIG. 5A, a first portion 141 of the instrument 140 is on a first side of the first view plane VP1 and a second portion 142 of the instrument 140 is on a second side of the first view plane VP1. In FIG. 5B, a third portion 143 of the instrument 140 is on a first side of the second view plane VP2 and a fourth portion 144 of the instrument 140 is on a second side of the second view plane VP2. In the example, the first and second portions 141, 142 of the instrument 140 are distinct from one another and the third and fourth portions 143, 144 of the instrument 140 are also distinct from one another, however, the third and fourth portions 143, 144 may not be distinct from the first and second portions 141, 142. In other words, at least one of the first and second portions 141, 142 may include some of at least one of the third and fourth portions 143, 144, and vice versa.

In FIG. 6A, the GUI 150 is illustrated showing slice representations of the spine of the patient along with the instrument representation 140R overlaid on the slice representations according to the spatial relationship shown in FIGS. 4-5B. First, the axial representation of the spine is shown in the second window 154 of the GUI 150, a first portion 141R of the instrument representation 140R is shown overlaid on the axial representation according to a first visual characteristic, and a second portion 142R of the instrument representation 140R is shown overlaid on the axial representation according to a second visual characteristic. Second, a sagittal representation of the spine is shown in the first window 152 of the GUI 150, a third portion 143R of the instrument representation 140R is shown overlaid on the sagittal representation according to the first visual characteristic, and a fourth portion 144R of the instrument representation 140R is shown overlaid on the sagittal representation according to the second visual characteristic. In FIG. 6A, the second and fourth portions 142R, 144R of the instrument representation 140R are shown with hatched lines to illustrate a different visual characteristic (e.g., color) from the first and third portions 141R, 143R. FIG. 6B is similar to FIG. 6A, except the second and fourth portions 142R, 144R of the instrument representation 140R are hidden and not overlaid on the slice representations.

Referring to FIGS. 7A-8B, an example of overlaying an implant representation 145R on the slice representation of the patient according to the method 200 is illustrated. In the illustrated implementation, the instrument representation 140R is also overlaid on the slice representation of the patient, but the implant representation 145R may instead be overlaid without the instrument representation 140R. FIGS. 7A and 7B show the spatial relationship between the instrument 140 and the first and second view planes VP1, VP2. In FIG. 7A, the implant 145 is entirely on the first side of the first view plane VP1. Thus, the system 100 may identify a first portion 146 of the implant 145 but not a second portion of the implant 145. In FIG. 7B, a third portion 148 of the implant 145 is on the first side of the second view plane VP2, and a fourth portion 149 of the implant 145 is on the second side of the second view plane VP2.

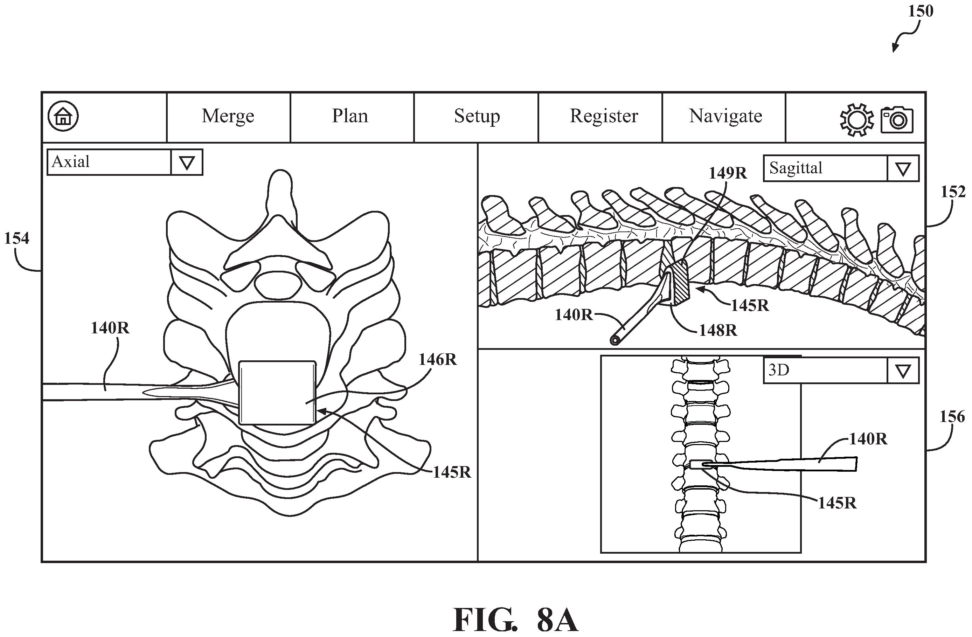

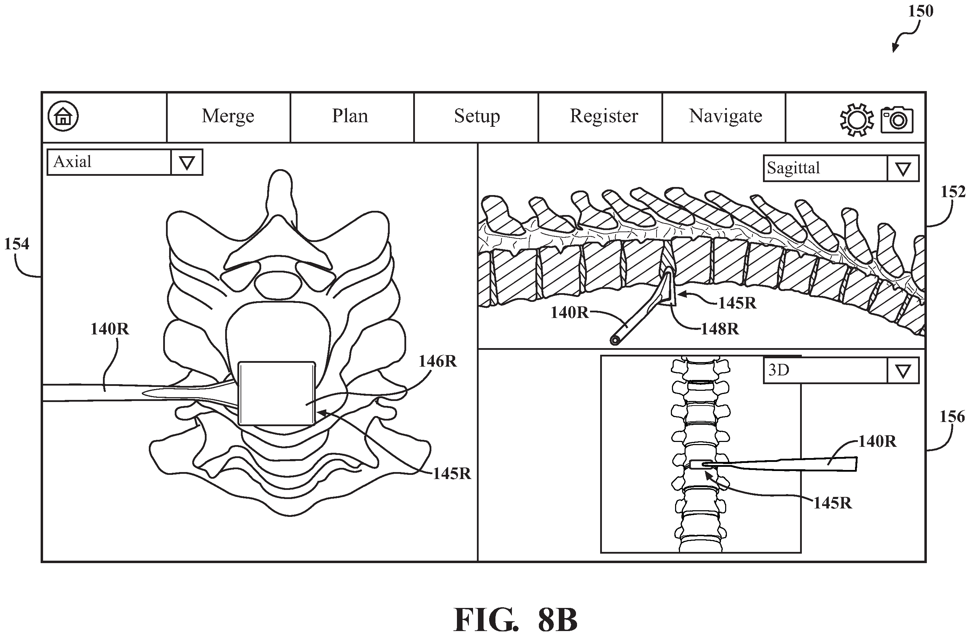

Turning to FIGS. 8A and 8B, the GUI 150 is illustrated showing slice representations of the spine of the patient along with the implant representation 145R overlaid on the slice representations according to the spatial relationship shown in FIGS. 7A and 7B. In the implementation of FIG. 8A, the GUI 150 is illustrated as showing a first portion 146R and a third portion 148R of the implant representation 145R overlaid according to a first visual characteristic and relative to axial and sagittal representations of the spine of the patient, respectively. Further, the fourth portion 149R of the implant representation 145R is overlaid according to a second visual characteristic and relative to the sagittal representation of the spine of the patient. In the implementation of FIG. 8B, the GUI 150 is shown with the first portion 146 and the first portion 148R of the implant representation 145R overlaid on the representations of the spine of the patient, while the fourth portion 149R of the implant representation 145R is hidden from view.

In the scenario depicted in FIGS. 7A-8B, differently from the example shown in FIGS. 4-6B, the implant 145 is entirely on the first side of the view plane corresponding to the axial slice representation. Thus, a second portion of the implant representation was not found by the controller 122 and the entirety of the implant representation 145R is shown according to the first visual characteristic relative to the axial representation. In other examples, the controller 122 may identify a second portion of the implant 145 which is on a second side of the view plane that corresponds to the axial slice representation, and a second portion of the implant representation 145R may be shown according to the second visual characteristic.

The systems and methods described herein may be partially or fully implemented as instructions stored on a non-transitory computer readable medium. The term computer-readable medium, as used herein, does not encompass transitory electrical or electromagnetic signals propagating through a medium (such as on a carrier wave); the term computer-readable medium may therefore be considered tangible and non-transitory. Non-limiting examples of a non-transitory computer-readable medium are nonvolatile memory circuits (such as a flash memory circuit, an erasable programmable read-only memory circuit, or a mask read-only memory circuit), volatile memory circuits (such as a static random access memory circuit or a dynamic random access memory circuit), magnetic storage media (such as an analog or digital magnetic tape or a hard disk drive), and optical storage media (such as a CD, a DVD, or a Blu-ray Disc).

The systems and methods described herein may be partially or fully implemented as computer program products. The computer program products may include processor-executable instructions that are stored on at least one non-transitory computer-readable medium. The computer program products may also include or rely on stored data. The computer program products may encompass a basic input/output system (BIOS) that interacts with hardware of the special purpose computer, device drivers that interact with particular devices of the special purpose computer, one or more operating systems, user applications, background services, background applications, etc. The computer program products may include: (i) descriptive text to be parsed, such as HTML (hypertext markup language), XML (extensible markup language), or JSON (JavaScript Object Notation), (ii) assembly code, (iii) object code generated from source code by a compiler, (iv) source code for execution by an interpreter, (v) source code for compilation and execution by a just-in-time compiler, etc. As examples only, source code may be written using syntax from languages including C, C++, C#, Objective-C, Swift, Haskell, Go, SQL, R, Lisp, Java^{®}, Fortran, Perl, Pascal, Curl, OCaml, JavaScript^{®}, HTML5 (Hypertext Markup Language 5th revision), Ada, ASP (Active Server Pages), PHP (PHP: Hypertext Preprocessor), Scala, Eiffel, Smalltalk, Erlang, Ruby, Flash^{®}, Visual Basic^{®}, Lua, MATLAB, SIMULINK, and Python^{®}.

Several embodiments have been discussed in the foregoing description. However, the implementations discussed herein are not intended to be exhaustive or limit the filter assembly to any particular form factor. The terminology which has been used is intended to be in the nature of words of description rather than of limitation. Many modifications and variations are possible in light of the above teachings and the system may be practiced otherwise than as specifically described.

Certain implementations may be described with reference to the following exemplary clauses:
Clause A1. A surgical navigation system (110) comprising: a localizer (112) configured to track movement of a tracked object (140, 145) relative to a patient, the tracked object (140, 145) including at least one of a surgical instrument (140) and an implant (145); a display (114) configured to show a representation (140R, 145R) of the tracked object (140, 145) relative to a representation (152, 154) of the patient; and a controller (122) in communication with the localizer (112) and the display (114), the controller (122) configured to: receive volumetric image data representative of the patient and defined relative to a first coordinate system; determine a pose of the tracked object (140, 145) relative to a second coordinate system based on signals received from the localizer (112); register the first coordinate system to the second coordinate system; determine a pose of a view plane (VP1, VP2) relative to the second coordinate system; cause the display (114) to show a slice representation (152, 154) of the patient based on the pose of the view plane (VP1, VP2); determine a spatial relationship between the tracked object (140, 145) and the view plane (VP1, VP2); identify a first portion (141, 146) of the tracked object (140, 145) that is on a first side of the view plane (VP1, VP2) and a second portion (142, 149) of the tracked object (140, 145) that is on a second side of the view plane (VP1, VP2) based on the spatial relationship; cause the display (114) to: overlay a first portion (141R, 146R) of the representation (140R, 145R) of the tracked object (140, 145) on the slice representation (152, 154) of the patient based on the spatial relationship; and hide a second portion (142R, 149R) of the representation (140R, 145R) of the tracked object (140, 145) such that the second portion is not overlaid on the slice representation (152, 154). A2. The surgical navigation system (110) of clause A1, wherein the tracked object (140, 145) comprises a surgical instrument (140). A3. The surgical navigation system (110) of clause A1, wherein the tracked object (140, 145) comprises an implant (145). A4. The surgical navigation system (110) of clause A3, wherein the implant (145) is coupled to the surgical instrument (140). A5. The surgical navigation system (110) of clause A1, wherein the representation (140R, 145R) of the tracked object (140, 145) is based on a three-dimensional model of the tracked object (140, 145). A6. The surgical navigation system (110) of clause A1, wherein the controller (122) is further configured to unhide at least a part of the second portion (142R, 149R) of the representation (140R, 145R) based on whether the second portion (142, 149) of the tracked object (140, 145) includes a critical portion of the tracked object (140, 145). A7. The surgical navigation system (110) of clause A1, wherein the controller (122) is configured to: cause the display (114) to overlay the first portion (141R, 146R) of the representation (140R, 145R) of the tracked object (140, 145) according to a first visual characteristic; determine whether the second portion (142, 149) of the tracked object (140, 145) includes a critical portion of the tracked object (140, 145); and cause the display (114) to overlay a representation (140R, 145R) of the critical portion on the slice representation (152, 154) of the patient according to a second visual characteristic that is different from the first visual characteristic. A8. The surgical navigation system (110) of clause A7, wherein the first and second visual characteristics each include at least one of: an opacity, transparency, color, outline, or shading of the corresponding portion of the representation (140R, 145R). A9. The surgical navigation system (110) of clause A1, wherein the controller (122) is configured to determine the pose of the view plane (VP1, VP2) based on a position of the tracked object (140, 145). A10. The surgical navigation system (110) of clause A1, wherein: the slice representation (152, 154) is a digitally reconstructed radiograph; and the controller (122) is configured to generate the digitally reconstructed radiograph based on the pose of the view plane (VP1, VP2). A11. The surgical navigation system (110) of clause A1, wherein: the view plane (VP1, VP2) is a first view plane (VP1); and the controller (122) is further configured to: determine a pose of a second view plane (VP2) relative to the second coordinate system, the second view plane (VP2) being orthogonal to the first view plane (VP1); cause the display (114) to present a second slice representation (152, 154) of the patient based on the pose of the second view plane (VP2); determine a spatial relationship between the tracked object (140, 145) and the second view plane (VP2); identify portions (143, 144, 148, 149) of the tracked object (140, 145) on opposite sides of the second view plane (VP2); and cause the display (114) to: overlay a portion (143R, 148R) of the representation (140R, 145R) of the tracked object (140, 145) corresponding to one side of the second view plane (VP2); and hide a portion (144R, 149R) of the representation (140R, 145R) of the tracked object (140, 145) corresponding to the other side.A12. The surgical navigation system (110) of clause A11, wherein: the first slice representation (154) is an axial slice representation of the patient; and the second slice representation (152) is a sagittal slice representation of the patient. A13. The surgical navigation system (110) of clause A2, wherein: the surgical instrument (140) includes an implant (145); and the controller (122) is configured to: determine a spatial relationship between the implant (145) and the view plane (VP1, VP2); identify portions (146, 148, 149) of the implant (145) on opposite sides of the view plane (VP1, VP2); and cause the display (114) to: overlay a portion (146R, 148R) of the representation (145R) of the implant (145) corresponding to one side of the view plane (VP1, VP2); and hide a portion (149R) of the representation (145R) of the implant (145) corresponding to the other side. A14. A non-transitory computer-readable medium having instructions stored thereon that, when executed by one or more processors of a surgical navigation system (110), cause the one or more processors to: receive volumetric image data representative of a patient and defined relative to a first coordinate system; determine a pose of a tracked object (140, 145) relative to a second coordinate system based on signals received from a localizer (112) of the surgical navigation system (110), the tracked object (140, 145) including at least one of a surgical instrument (140) and an implant (145); register the first coordinate system to the second coordinate system; determine a pose of a view plane (VP1, VP2) relative to the second coordinate system; cause a display (114) of the surgical navigation system (110) to present a slice representation (152, 154) of the patient based on the pose of the view plane (VP1, VP2); determine a spatial relationship between the tracked object (140, 145) and the view plane (VP1, VP2); identify a first portion (141, 146) of the tracked object (140, 145) that is on a first side of the view plane (VP1, VP2) and a second portion (142, 149) of the tracked object (140, 145) that is on a second side of the view plane (VP1, VP2) based on the spatial relationship; and cause the display (114) to: overlay a first portion (141R, 146R) of the representation (140R, 145R) of the tracked object (140, 145) on the slice representation (152, 154) of the patient based on the spatial relationship; and hide a second portion (142R, 149R) of the representation (140R, 145R) of the tracked object (140, 145) such that the second portion is not overlaid on the slice representation (152, 154).
Clause B1. A surgical navigation system (110) comprising: a localizer (112) configured to track movement of a surgical instrument (140) relative to a patient; a display (114) configured to present a representation (140R) of the surgical instrument (140) relative to a representation of the patient; a controller (122) in communication with the localizer (112) and the display (114), the controller (122) configured to: receive volumetric image data representative of the patient and defined relative to a first coordinate system; determine a pose of the surgical instrument (140) relative to a second coordinate system based on signals received from the localizer (112); register the first coordinate system to the second coordinate system; determine a pose of a view plane relative to the second coordinate system; cause the display (114) to present a slice representation of the patient based on the pose of the view plane; determine a spatial relationship between the surgical instrument (140) and the view plane; identify a first portion of the surgical instrument (140) that is on a first side of the view plane and a second portion of the surgical instrument (140) that is on a second side of the view plane based on the spatial relationship; cause the display (114) to overlay a first portion of the representation (140R) of the surgical instrument (140) on the slice representation of the patient based on the spatial relationship and according to a first visual characteristic, the first portion of the representation (140R) corresponding to the first portion of the surgical instrument (140); and cause the display (114) to overlay a second portion of the representation (140R) of the surgical instrument (140) on the slice representation of the patient based on the spatial relationship and according to a second visual characteristic that is different from the first visual characteristic, the second portion of the representation (140R) corresponding to the second portion of the surgical instrument (140). B2. The surgical navigation system (110) of clause B1, wherein the representation (140R) of the surgical instrument (140) is based on a three-dimensional model that is representative of the surgical instrument (140). B3. The surgical navigation system (110) of any preceding clause, wherein the first and second visual characteristics each include at least one of the following: an opacity of the corresponding portion of the representation (140R), a transparency of the corresponding portion of the representation (140R), a color of the corresponding portion of the representation (140R), an outline of the corresponding portion of the representation (140R), and a shading of the corresponding portion of the representation (140R). B4. The surgical navigation system (110) of any preceding clause, wherein the first visual characteristic includes a first color and the second visual characteristic is a second color different than the first color. B5. The surgical navigation system of any preceding clause, wherein the first visual characteristic includes a first opacity and the second visual characteristic includes a second opacity different than the first opacity. B6. The surgical navigation system (110) of any preceding clause, wherein the first visual characteristic is a filled shape and the second visual characteristic is a shape outline. B7.The surgical navigation system (110) of any preceding clause, wherein the controller (122) is further configured to select the second visual characteristic based on whether the second portion of the surgical instrument (140) includes a critical portion of the surgical instrument (140). B8.The surgical navigation system (110) of any preceding clause, wherein the controller (122) is further configured to: determine whether the second portion of the surgical instrument (140) includes a critical portion of the surgical instrument (140); and cause the display (114) to overlay a critical portion of the representation (140R) of the surgical instrument (140) on the slice representation of the patient based on the spatial relationship and according to a third visual characteristic that is different from the first visual characteristic and the second visual characteristic. B9. The surgical navigation system (110) of any preceding clause, wherein the controller (122) is configured to determine the pose of the view plane based on a position of the surgical instrument (140). B10. The surgical navigation system (110) of any preceding clause, wherein: the view plane is realized as a first view plane; the slice representation is realized as a first slice representation of the patient; and the controller (122) is configured to: determine a pose of a second view plane relative to the second coordinate system, the second view plane being orthogonal to the first view plane; cause the display (114) to present a second slice representation of the patient based on the pose of the second view plane, the first and second slice representations being orthogonal representations of the patient; determine a spatial relationship between surgical instrument (140) and the second view plane; identify a third portion of the surgical instrument (140) that is on a first side of the second view plane and a fourth portion of the surgical instrument (140) that is on a second side of the second view plane based on the spatial relationship; cause the display (114) to overlay a third portion of the representation (140R) of the surgical instrument (140) on the second slice representation of the patient based on the spatial relationship and according to the first visual characteristic, the third portion of the representation (140R) corresponding to the third portion of the surgical instrument (140); and cause the display (114) to overlay the fourth portion of the representation (140R) of the surgical instrument (140) on the second slice representation of the patient based on the spatial relationship and according to the second visual characteristic that is different from the first visual characteristic, the fourth portion of the representation (140R) corresponding to the fourth portion of the surgical instrument (140). B11. The surgical navigation system (110) of clause B10, wherein the first slice representation is an axial slice representation of the patient and the second slice representation is a sagittal slice representation of the patient. B12.The surgical navigation system (110) of any preceding clause, wherein: the surgical instrument (140) includes an implant (145); and the controller (122) is further configured to cause the display (114) to overlay a representation (145R) of the implant (145) on the slice representation of the patient based on the spatial relationship. B13. The surgical navigation system (110) of clause B12, wherein the controller (122) is configured to: identify a first portion of the implant (145) that is on the first side of the view plane and a second portion of the implant (145) that is on the second side of the view plane based on the spatial relationship; cause the display (114) to overlay a first portion of the representation (145R) of the implant (145) on the slice representation of the patient based on the spatial relationship and according to the first visual characteristic, the first portion of the representation (145R) corresponding to the first portion of the implant (145); and cause the display (114) to overlay a second portion of the representation (145R) of the implant (145) on the slice representation of the patient based on the spatial relationship and according to the second visual characteristic that is different from the first visual characteristic, the second portion of the representation (145R) corresponding to the second portion of the implant (145). B14. A non-transitory computer-readable medium having instructions stored thereon that, when executed by one or more processors of a surgical navigation system (110), cause the one or more processors to: receive volumetric image data representative of a patient and defined relative to a first coordinate system; determine a pose of a surgical instrument (140) relative to a second coordinate system based on signals received from a localizer (112) of the surgical navigation system (110); register the first coordinate system to the second coordinate system; determine a pose of a view plane relative to the second coordinate system; cause a display (114) of the surgical navigation system (110) to present a slice representation of the patient based on the pose of the view plane; determine a spatial relationship between the surgical instrument (140) and the view plane; identify a first portion of the surgical instrument (140) that is on a first side of the view plane and a second portion of the surgical instrument (140) that is on a second side of the view plane based on the spatial relationship; cause the display (114) to overlay a first portion of a representation (140R) of the surgical instrument (140) on the slice representation of the patient based on the spatial relationship and according to a first visual characteristic, the first portion of the representation (140R) corresponding to the first portion of the surgical instrument (140); and cause the display (114) to overlay a second portion of the representation (140R) of the surgical instrument (140) on the slice representation of the patient based on the spatial relationship and according to a second visual characteristic that is different from the first visual characteristic, the second portion of the representation (140R) corresponding to the second portion of the surgical instrument (140).
Clause C1. A surgical navigation system (110) comprising: a localizer (112) configured to track movement of a surgical instrument (140) relative to a patient; a display (114) configured to present a representation (140R) of the surgical instrument (140) relative to a representation of the patient; a controller (122) in communication with the localizer (112) and the display (114), the controller (122) configured to: receive volumetric image data representative of the patient and defined relative to a first coordinate system; determine a pose of the surgical instrument (140) relative to a second coordinate system based on signals received from the localizer (112); register the first coordinate system to the second coordinate system; determine a pose of a view plane relative to the second coordinate system; cause the display (114) to present a slice representation of the patient based on the pose of the view plane; determine a spatial relationship between the surgical instrument (140) and the view plane; identify a first portion of the surgical instrument (140) that is on a first side of the view plane and a second portion of the surgical instrument (140) that is on a second side of the view plane based on the spatial relationship; cause the display (114) to overlay a first portion of the representation (140R) of the surgical instrument (140) on the slice representation of the patient based on the spatial relationship; and cause the display (114) to hide a second portion of the representation (140R) of the surgical instrument (140) such that the second portion is not overlaid on the slice representation. C2. The surgical navigation system (110) of clause C1, wherein the representation (140R) of the surgical instrument (140) is based on a three-dimensional model that is representative of the surgical instrument (140). C3. The surgical navigation system (110) of any preceding clause, wherein the controller (122) is further configured to unhide at least a part of the second portion of the representation (140R) based on whether the second portion of the surgical instrument (140) includes a critical portion of the surgical instrument (140). C4. The surgical navigation system (110) of any preceding clause, wherein the controller (122) is configured to: cause the display (114) to overlay the first portion of the representation (140R) of the surgical instrument (140) according to a first visual characteristic; determine whether the second portion of the surgical instrument (140) includes a critical portion of the surgical instrument (140); and cause the display (114) to overlay a critical portion of the representation (140R) of the surgical instrument (140) on the slice representation of the patient based on the spatial relationship and according to a second visual characteristic that is different from the first visual characteristic. C5. The surgical navigation system (110) of clause C4, wherein the first and second visual characteristics each include at least one of the following: an opacity of the corresponding portion of the representation (140R), a transparency of the corresponding portion of the representation (140R), a color of the corresponding portion of the representation (140R), an outline of the corresponding portion of the representation (140R), and a shading of the corresponding portion of the representation (140R). C6. The surgical navigation system (110) of any preceding clause, wherein the controller (122) is configured to determine the pose of the view plane based on a position of the surgical instrument (140). C7. The surgical navigation system (110) of any preceding clause, wherein: the slice representation is a digitally reconstructed radiograph, and the controller (122) is configured to generate the digitally reconstructed radiograph based on the pose of the view plane. C8.The surgical navigation system (110) of any preceding clause, wherein: the view plane is realized as a first view plane; the slice representation is realized as a first slice representation of the patient; and the controller (122) is configured to: determine a pose of a second view plane relative to the second coordinate system, the second view plane being orthogonal to the first view plane; cause the display (114) to present a second slice representation of the patient based on the pose of the second view plane, the first and second slice representations being orthogonal representations of the patient; determine a spatial relationship between surgical instrument (140) and the second view plane; identify a third portion of the surgical instrument (140) that is on a first side of the second view plane and a fourth portion of the surgical instrument (140) that is on a second side of the second view plane based on the spatial relationship; cause the display (114) to overlay a third portion of the representation (140R) of the surgical instrument (140) on the second slice representation of the patient based on the spatial relationship, the third portion of the representation (140R) corresponding to the third portion of the surgical instrument (140); and cause the display (114) to hide the fourth portion of the representation (140R) of the surgical instrument (140) such that the fourth portion is not overlaid on the second slice representation. C9. The surgical navigation system (110) of clause C8, wherein the first slice representation is an axial slice representation of the patient and the second slice representation is a sagittal slice representation of the patient. C10. A non-transitory computer-readable medium having instructions stored thereon that, when executed by one or more processors of a surgical navigation system (110), cause the one or more processors to: receive volumetric image data representative of a patient and defined relative to a first coordinate system; determine a pose of a surgical instrument (140) relative to a second coordinate system based on signals received from a localizer (112) of the surgical navigation system (110); register the first coordinate system to the second coordinate system; determine a pose of a view plane relative to the second coordinate system; cause a display (114) of the surgical navigation system (110) to present a slice representation of the patient based on the pose of the view plane; determine a spatial relationship between the surgical instrument (140) and the view plane; identify a first portion of the surgical instrument (140) that is on a first side of the view plane and a second portion of the surgical instrument (140) that is on a second side of the view plane based on the spatial relationship; cause the display (114) to overlay a first portion of a representation (140R) of the surgical instrument (140) on the slice representation of the patient based on the spatial relationship, the first portion of the representation (140R) corresponding to the first portion of the surgical instrument (140); and cause the display (114) to hide a second portion of the representation (140R) of the surgical instrument (140) such that the second portion is not overlaid on the slice representation, the second portion of the representation (140R) corresponding to the second portion of the surgical instrument (140).
Clause D1. A surgical navigation system (110) comprising: a localizer (112) configured to track movement of a surgical instrument (140) relative to a patient; a display (114) configured to present a representation (145R) of an implant (145) coupled to the surgical instrument (140) relative to a representation of the patient; a controller (122) in communication with the localizer (112) and the display (114), the controller (122) configured to: receive volumetric image data representative of the patient and defined relative to a first coordinate system; determine a pose of the surgical instrument (140) relative to a second coordinate system based on signals received from the localizer (112); register the first coordinate system to the second coordinate system; determine a pose of a view plane relative to the second coordinate system; cause the display (114) to present a slice representation of the patient based on the pose of the view plane; determine a spatial relationship between the implant (145) and the view plane; identify a first portion of the implant (145) that is on a first side of the view plane and a second portion of the implant (145) that is on a second side of the view plane based on the spatial relationship; cause the display (114) to overlay a first portion of the representation (145R) of the implant (145) on the slice representation of the patient based on the spatial relationship and according to a first visual characteristic, the first portion of the representation (145R) corresponding to the first portion of the implant (145); and cause the display (114) to overlay a second portion of the representation (145R) of the implant (145) on the slice representation of the patient based on the spatial relationship and according to a second visual characteristic that is different from the first visual characteristic, the second portion of the representation (145R) corresponding to the second portion of the implant (145). D2. The surgical navigation system (110) of clause D1, wherein the representation (145R) of the implant (145) is based on a three-dimensional implant (145) model that is representative of the implant (145). D3.The surgical navigation system (110) of any preceding clause, wherein the first and second visual characteristics each include at least one of the following: an opacity of the corresponding portion of the representation (145R), a transparency of the corresponding portion of the representation (145R), a color of the corresponding portion of the representation (145R), an outline of the corresponding portion of the representation (145R), and a shading of the corresponding portion of the representation (145R). D4. The surgical navigation system (110) of any preceding clause, wherein the first visual characteristic includes a first color and the second visual characteristic is a second color different than the first color. D5. The surgical navigation system (110) of any preceding clause, wherein the first visual characteristic includes a first opacity and the second visual characteristic includes a second opacity different than the first opacity. D6. The surgical navigation system (110) of any preceding clause, wherein the first visual characteristic is a filled shape and the second visual characteristic is a shape outline. D7. The surgical navigation system (110) of any preceding clause, wherein the controller (122) is further configured to select the second visual characteristic based on whether the second portion of the implant (145) includes a critical portion of the implant (145). D8.The surgical navigation system (110) of any preceding clause, wherein the controller (122) is further configured to: determine whether the second portion of the implant (145) includes a critical portion of the implant (145); and cause the display (114) to overlay a critical portion of the representation (145R) of the implant (145) on the slice representation of the patient based on the spatial relationship and according to a third visual characteristic that is different from the first visual characteristic and the second visual characteristic. D9. The surgical navigation system (110) of any preceding clause, wherein the controller (122) is configured to determine the pose of the view plane based on at least one of a position of the surgical instrument (140) and a position of the implant (145). D10. The surgical navigation system (110) of any preceding clause, wherein: the slice representation is a digitally reconstructed radiograph, and the controller (122) is configured to generate the digitally reconstructed radiograph based on the pose of the view plane. D11. The surgical navigation system (110) of any preceding clause, wherein: the view plane is realized as a first view plane; the slice representation is realized as a first slice representation of the patient; and the controller (122) is configured to: determine a pose of a second view plane relative to the second coordinate system, the second view plane being orthogonal to the first view plane; determine a spatial relationship between the implant (145) and the second view plane; cause the display (114) to present a second slice representation of the patient based on the pose of the second view plane, the first and second slice representations being orthogonal representations of the patient; identify a third portion of the implant (145) that is on a first side of the second view plane and a fourth portion of the implant (145) that is on a second side of the second view plane based on the spatial relationship; cause the display (114) to overlay the third portion of the representation (145R) of the implant (145) on the second slice representation of the patient based on the spatial relationship and according to the first visual characteristic; and cause the display (114) to overlay the fourth portion of the representation (145R) of the implant (145) on the second slice representation of the patient based on the spatial relationship and according to the second visual characteristic that is different from the first visual characteristic. D12. The surgical navigation system (110) of clause D11, wherein the first slice representation is an axial slice representation of the patient and the second slice representation is a sagittal slice representation of the patient. D13. A non-transitory computer-readable medium having instructions stored thereon that, when executed by one or more processors of a surgical navigation system (110), cause the one or more processors to: receive volumetric image data representative of a patient and defined relative to a first coordinate system; determine a pose of a surgical instrument (140) relative to a second coordinate system based on signals received from a localizer (112) of the surgical navigation system (110); register the first coordinate system to the second coordinate system; determine a pose of a view plane relative to the second coordinate system; cause a display (114) of the surgical navigation system (110) to present a slice representation of the patient based on the pose of the view plane; determine a spatial relationship between an implant (145) coupled to the surgical instrument (140) and the view plane; identify a first portion of the implant (145) that is on a first side of the view plane and a second portion of the implant (145) that is on a second side of the view plane based on the spatial relationship; cause the display (114) to overlay a first portion of a representation (145R) of the implant (145) on the slice representation of the patient based on the spatial relationship and according to a first visual characteristic, the first portion of the representation (145R) corresponding to the first portion of the implant (145); and cause the display (114) to overlay a second portion of the representation (145R) of the implant (145) on the slice representation of the patient based on the spatial relationship and according to a second visual characteristic that is different from the first visual characteristic, the second portion of the representation (145R) corresponding to the second portion of the implant (145).
Clause E1. A surgical navigation system (110) comprising: a localizer (112) configured to track movement of a surgical instrument (140) relative to a patient; a display (114) configured to present a representation (145R) of an implant (145) coupled to the surgical instrument (140) relative to a representation of the patient; and a controller (122) in communication with the localizer (112) and the display (114), the controller (122) configured to: receive volumetric image data representative of the patient and defined relative to a first coordinate system; determine a pose of the surgical instrument (140) relative to a second coordinate system based on signals received from the localizer (112); register the first coordinate system to the second coordinate system; determine a pose of a view plane relative to the second coordinate system; cause the display (114) to present a slice representation of the patient based on the pose of the view plane; determine a spatial relationship between the implant (145) and the view plane; identify a first portion of the implant (145) that is on a first side of the view plane and a second portion of the implant (145) that is on a second side of the view plane based on the spatial relationship; cause the display (114) to overlay a first portion of the representation (145R) of the implant (145) on the slice representation of the patient based on the spatial relationship; and cause the display (114) to hide a second portion of the representation (145R) of the implant (145) such that the second portion is not overlaid on the slice representation. E2. The surgical navigation system (110) of clause E1, wherein the representation (145R) of the implant (145) is based on a three-dimensional implant (145) model that is representative of the implant (145). E3. The surgical navigation system (110) of any preceding clause, wherein the controller (122) is further configured to unhide at least a part of the second portion of the representation (145R) based on whether the second portion of the implant (145) includes a critical portion of the implant (145). E4.The surgical navigation system (110) of any preceding clause, wherein the controller (122) is configured to: cause the display (114) to overlay the first portion of the representation (145R) of the implant (145) according to a first visual characteristic; determine whether the second portion of the implant (145) includes a critical portion of the implant (145); and cause the display (114) to overlay a critical portion of the representation (145R) of the implant (145) on the slice representation of the patient based on the spatial relationship and according to a second visual characteristic that is different from the first visual characteristic. E5. The surgical navigation system (110) of clause E4, wherein the first and second visual characteristics each include at least one of the following: an opacity of the corresponding portion of the representation (145R), a transparency of the corresponding portion of the representation (145R), a color of the corresponding portion of the representation (145R), an outline of the corresponding portion of the representation (145R), and a shading of the corresponding portion of the representation (145R). E6. The surgical navigation system (110) of any preceding clause, wherein the controller (122) is configured to determine the pose of the view plane based on a position of the surgical instrument (140). E7. The surgical navigation system (110) of any preceding clause, wherein: the slice representation is a digitally reconstructed radiograph, and the controller (122) is configured to generate the digitally reconstructed radiograph based on the pose of the view plane. E8.The surgical navigation system (110) of any preceding clause, wherein: the view plane is realized as a first view plane; the slice representation is realized as a first slice representation of the patient; and the controller (122) is configured to: determine a pose of a second view plane relative to the second coordinate system, the second view plane being orthogonal to the first view plane; cause the display (114) to present a second slice representation of the patient based on the pose of the second view plane, the first and second slice representations being orthogonal representations of the patient; determine a spatial relationship between the implant (145) and the second view plane; identify a third portion of the implant (145) that is on a first side of the second view plane and a fourth portion of the implant (145) that is on a second side of the second view plane based on the spatial relationship; cause the display (114) to overlay a third portion of the representation (145R) of the implant (145) on the second slice representation of the patient based on the spatial relationship, the third portion of the representation (145R) corresponding to the third portion of the implant (145); and cause the display (114) to hide the fourth portion of the representation (145R) of the implant (145) such that the fourth portion is not overlaid on the second slice representation. E9. The surgical navigation system (110) of clause E8, wherein the first slice representation is an axial slice representation of the patient and the second slice representation is a sagittal slice representation of the patient. E10. A non-transitory computer-readable medium having instructions stored thereon that, when executed by one or more processors of a surgical navigation system (110), cause the one or more processors to: receive volumetric image data representative of a patient and defined relative to a first coordinate system; determine a pose of a surgical instrument (140) relative to a second coordinate system based on signals received from a localizer (112) of the surgical navigation system (110); register the first coordinate system to the second coordinate system; determine a pose of a view plane relative to the second coordinate system; cause a display (114) of the surgical navigation system (110) to present a slice representation of the patient based on the pose of the view plane; determine a spatial relationship between an implant (145) coupled to the surgical instrument (140) and the view plane; identify a first portion of the implant (145) that is on a first side of the view plane and a second portion of the implant (145) that is on a second side of the view plane based on the spatial relationship; cause the display (114) to overlay a first portion of a representation (145R) of the implant (145) on the slice representation of the patient based on the spatial relationship; and cause the display to hide a second portion of the representation (145R) of the implant (145) such that the second portion is not overlaid on the slice representation.

## Claims

1. A surgical navigation system (110) comprising:
a localizer (112) configured to track movement of a tracked object (140, 145) relative to a patient, the tracked object (140, 145) including at least one of a surgical instrument (140) and an implant (145);
a display (114) configured to present a representation (140R, 145R) of the tracked object (140, 145) relative to a representation (152, 154) of the patient; and
a controller (122) in communication with the localizer (112) and the display (114), the controller (122) configured to:
receive volumetric image data representative of the patient and defined relative to a first coordinate system;
determine a pose of the tracked object (140, 145) relative to a second coordinate system based on signals received from the localizer (112);
register the first coordinate system to the second coordinate system;
determine a pose of a view plane (VP1, VP2) relative to the second coordinate system;
cause the display (114) to present a slice representation (152, 154) of the patient based on the pose of the view plane (VP1, VP2);
determine a spatial relationship between the tracked object (140, 145) and the view plane (VP1, VP2);
identify a first portion (141, 146) of the tracked object (140, 145) that is on a first side of the view plane (VP1, VP2) and a second portion (142, 149) of the tracked object (140, 145) that is on a second side of the view plane (VP1, VP2) based on the spatial relationship;
cause the display (114) to overlay a first portion (141R, 146R) of the representation (140R, 145R) of the tracked object (140, 145) on the slice representation (152, 154) of the patient based on the spatial relationship and according to a first visual characteristic, the first portion (141R, 146R) of the representation (140R, 145R) corresponding to the first portion (141, 146) of the tracked object (140, 145); and
cause the display (114) to overlay a second portion (142R, 149R) of the representation (140R, 145R) of the tracked object (140, 145) on the slice representation (152, 154) of the patient based on the spatial relationship and according to a second visual characteristic that is different from the first visual characteristic, the second portion (142R, 149R) of the representation (140R, 145R) corresponding to the second portion (142, 149) of the tracked object (140, 145).

2. The surgical navigation system (110) of claim 1, wherein the tracked object (140, 145) comprises the surgical instrument (140).

3. The surgical navigation system (110) of claim 1, wherein:
the tracked object (140, 145) comprises the implant (145);
and optionally
the implant (145) is coupled to the surgical instrument (140).

4. The surgical navigation system (110) of any preceding claim, wherein the representation (140R, 145R) of the tracked object (140, 145) is based on a three-dimensional model of the tracked object (140, 145).

5. The surgical navigation system (110) of any preceding claim, wherein the first and second visual characteristics each include at least one of:
an opacity of the corresponding portion of the representation (140R, 145R),
a transparency of the corresponding portion of the representation (140R, 145R),
a color of the corresponding portion of the representation (140R, 145R),
an outline of the corresponding portion of the representation (140R, 145R), and
a shading of the corresponding portion of the representation (140R, 145R).

6. The surgical navigation system (110) of any preceding claim, wherein:
the first visual characteristic includes a first color; and
the second visual characteristic includes a second color different from the first color.

7. The surgical navigation system (110) of any preceding claim, wherein:
the first visual characteristic includes a first opacity; and
the second visual characteristic includes a second opacity different from the first opacity.

8. The surgical navigation system (110) of any preceding claim, wherein:
the first visual characteristic corresponds to a filled representation (140R, 145R); and
the second visual characteristic corresponds to an outline representation (140R, 145R).

9. The surgical navigation system (110) of any preceding claim, wherein the controller (122) is configured to select the second visual characteristic based on whether the second portion (142, 149) of the tracked object (140, 145) includes a critical portion of the tracked object (140, 145).

10. The surgical navigation system (110) of any preceding claim, wherein the controller (122) is further configured to:
determine whether the second portion (142, 149) of the tracked object (140, 145) includes a critical portion of the tracked object (140, 145); and
cause the display (114) to visually distinguish a representation of the critical portion according to a third visual characteristic different from the first visual characteristic and the second visual characteristic.

11. The surgical navigation system (110) of any preceding claim, wherein the controller (122) is configured to determine the pose of the view plane (VP1, VP2) based on a position of the tracked object (140, 145).

12. The surgical navigation system (110) of any preceding claim, wherein:
the slice representation (152, 154) is a digitally reconstructed radiograph; and
the controller (122) is configured to generate the digitally reconstructed radiograph based on the pose of the view plane (VP1, VP2).

13. The surgical navigation system (110) of any preceding claim, wherein:
the view plane (VP1, VP2) is a first view plane (VP1); and
the controller (122) is further configured to:
determine a pose of a second view plane (VP2) relative to the second coordinate system, the second view plane (VP2) being orthogonal to the first view plane (VP1);
cause the display (114) to present a second slice representation (152) of the patient based on the pose of the second view plane (VP2);
determine a spatial relationship between the tracked object (140, 145) and the second view plane (VP2);
identify portions of the tracked object (140, 145) on opposite sides of the second view plane (VP2); and
cause the display (114) to overlay corresponding portions of the representation (140R, 145R) of the tracked object (140, 145) on the second slice representation (152) of the patient according to different visual characteristics based on the spatial relationship;
and optionally.
the first slice representation (154) is an axial slice representation of the patient; and
the second slice representation (152) is a sagittal slice representation of the patient.

14. The surgical navigation system (110) of claim 2, wherein:
the surgical instrument (140) includes an implant (145); and
the controller (122) is further configured to cause the display (114) to overlay a representation (145R) of the implant (145) on the slice representation (152, 154) of the patient based on the spatial relationship;
and optionally,
the controller (122) is configured to apply the first and second visual characteristics to the implant (145) separate from application of visual characteristics to the surgical instrument (140).

15. A non-transitory computer-readable medium having instructions stored thereon that, when executed by one or more processors of a surgical navigation system (110), cause the one or more processors to:
receive volumetric image data representative of a patient and defined relative to a first coordinate system;
determine a pose of a tracked object (140, 145) relative to a second coordinate system based on signals received from a localizer (112) of the surgical navigation system (110), the tracked object (140, 145) including at least one of a surgical instrument (140) and an implant (145);
register the first coordinate system to the second coordinate system;
determine a pose of a view plane (VP1, VP2) relative to the second coordinate system;
cause a display (114) of the surgical navigation system (110) to present a slice representation (152, 154) of the patient based on the pose of the view plane (VP1, VP2);
determine a spatial relationship between the tracked object (140, 145) and the view plane (VP1, VP2);
identify a first portion (141, 146) of the tracked object (140, 145) that is on a first side of the view plane (VP1, VP2) and a second portion (142, 149) of the tracked object (140, 145) that is on a second side of the view plane (VP1, VP2) based on the spatial relationship;
cause the display (114) to overlay a first portion (141R, 146R) of a representation (140R, 145R) of the tracked object (140, 145) on the slice representation (152, 154) of the patient based on the spatial relationship and according to a first visual characteristic, the first portion (141R, 146R) of the representation (140R, 145R) corresponding to the first portion (141, 146) of the tracked object (140, 145); and
cause the display (114) to overlay a second portion (142R, 149R) of the representation (140R, 145R) of the tracked object (140, 145) on the slice representation (152, 154) of the patient based on the spatial relationship and according to a second visual characteristic that is different from the first visual characteristic, the second portion (142R, 149R) of the representation (140R, 145R) corresponding to the second portion (142, 149) of the tracked object (140, 145).
